Europäisches Patentamt

European Patent Office

Office européen des brevets

(11) Numéro de publication: **0 232 638**
**A1**

## (12) DEMANDE DE BREVET EUROPEEN

(21) Numéro de dépôt: 86402700.8

(22) Date de dépôt: 05.12.86

(51) Int. Cl.⁴: **A61N 1/32**

(30) Priorité: 10.12.85 FR 8518258

(43) Date de publication de la demande:
19.08.87 Bulletin 87/34

(84) Etats contractants désignés:
**DE IT SE**

(71) Demandeur: **C.G.R. MeV**
**551, route de la Minière**
**F-78530 Buc(FR)**

(72) Inventeur: **Convert, Guy**
**THOMSON-CSF SCPI 19, avenue de Messine**
**F-75008 Paris(FR)**
Inventeur: **Azam, Guy**
**THOMSON-CSF SCPI 19, avenue de Messine**
**F-75008 Paris(FR)**
Inventeur: **Mabire, Jean-Pierre**
**THOMSON-CSF SCPI 19, avenue de Messine**
**F-75008 Paris(FR)**
Inventeur: **Jasmin, Claude**
**THOMSON-CSF SCPI 19, avenue de Messine**
**F-75008 Paris(FR)**
Inventeur: **Sidi, Joel**
**THOMSON-CSF SCPI 19, avenue de Messine**
**F-75008 Paris(FR)**

(74) Mandataire: **Grynwald, Albert et al**
**THOMSON-CSF SCPI 19, avenue de Messine**
**F-75008 Paris(FR)**

(54) **Appareil de traitement par hyperthermie.**

(57) L'invention concerne un appareil de traitement par hyperthermie, particulièrement destiné à l'hyperthermie profonde. L'appareil de traitement (1) comprend au moins deux générateurs (G1, G2) fonctionnant à des fréquences (F1, F2) différentes de manière à éviter une corrélation de phase entre les ondes délivrées par les générateurs (G1, G2).

FIG_1

# APPAREIL DE TRAITEMENT PAR HYPERTHERMIE

L'invention concerne un appareil de traitement par hyperthermie, particulièrement bien adapté pour l'hyperthermie en profondeur, c'est-à-dire dans les cas où une zone à traiter d'un patient est située à un niveau relativement profond dans le corps du patient.

L'hyperthermie est un procédé qui consiste à chauffer des tissus biologiques vivants, par exemple par dissipassion d'une onde électromagnétique appliquée à une zone à traiter grâce à des applicateurs disposés au voisinage de cette zone. Ce procédé est utilisé dans le traitement de diverses maladies et notamment en cancérothérapie. Dans l'exemple de cette dernière application, il est souhaitable de chauffer les tissus à traiter à des températures de l'ordre de 44°C à 45°C, tout en évitant autant que possible, une élévation notable de la température des tissus sains environnants.

Un des problèmes posés est alors la localisation correcte de la zone chauffée par rapport à la zone à traiter. Cette condition étant généralement réalisée convenablement pour des traitements de tumeurs superficielles, ou peu profondes, les applicateurs posés sur le corps du patient étant dans ce cas au voisinage immédiat de la zone à traiter.

Par contre dans le cas du traitement des tumeurs profondes (intestins, prostate, ...), le problème de la localisation correcte de la puissance appliquée se pose avec beaucoup plus d'accuité, du fait notamment que la zone du patient comprise entre deux électrodes comprend également des régions saines ; le problème étant compliqué en outre par le fait que des températures de tissus cutanés, supérieurs à 44°C, sont en règle générale très mal supportées par un patient.

La technique la plus largement utilisée en hyperthermie profonde consiste à réaliser le chauffage par champ électromagnétique, à des fréquences suffisamment basses pour n'être pas limitées par les effets de peau, qui empêchent la pénétration de ces champs électromagnétiques quand les fréquences sont supérieures à par exemple 50 ou 60 MHZ. Les applicateurs comportent des électrodes, ou des cornets ou des guides d'onde, par l'intermédiaire desquels est appliquée la puissance électromagnétique. Schématiquement, la région chauffée est la région enclose par les électrodes, ou encore dans le cas d'un cornet ou d'un guide d'ondes, les dimensions de la région chauffée sont de l'ordre des dimensions transversales de l'applicateur. On peut ainsi, en principe, modifier la répartition de la puissance dissipée en jouant sur les dimensions et la position des électrodes. Cependant l'utilisation de ce procédé se heurte à des difficultés pratiques, du fait que dans une configuration donnée d'électrodes, la distribution de la puissance dissipée dépend des propriétés électriques des tissus à chauffer. On observe en outre que la répartition de la puissance dissipée peut varier très fortement pour des variations apparemment peu importantes de la nature des tissus à chauffer. Aussi, en pratique, pour obtenir un chauffage aussi satisfaisant que possible, le praticien est conduit à ajuster au cours même du traitement par hyperthermie, la distribution de la puissance dissipée, en fonction notamment d'informations communiqués par des sondes de température.

A cette fin, il est connu dans l'art antérieur, par une demande de brevet français N° 83 08727, d'utiliser des moyens générateurs haute fréquence comprenant au moins trois générateurs fonctionnant à une même fréquence et selon des phases relatives ajustables ; chaque générateur étant relié à une électrode. Cette configuration permet de localiser une zone plus fortement chauffée, dans un espace compris entre les trois électrodes. Un des inconvénients de cette disposition, dans laquelle des générateurs fonctionnent à une même fréquence avec des relations de phase bien définies entre eux, réside en ce que des effets d'interférences du champ peuvent se produire dans la zone à chauffer, de sorte que la puissance moyenne dissipée dans un volume donné, peut être supérieure ou inférieure, à la somme des puissances moyennes dissipées par chacun des générateurs fonctionnant seuls. Il peut en résulter des différences de température importantes entre deux points relativement proches, dont la correction peut conduire à des réglages longs et délicats.

La présente invention concerne un appareil d'hyperthermie, particulièrement bien adapté à l'hyperthermie profonde, permettant de manière plus précise que dans l'art antérieur d'adapter la configuration de la zone chauffée à la zone à traiter, et permettant d'une manière souple et sûre soit d'augmenter la puissance dissipée dans la tumeur à traiter avec une incidence négligeable au niveau des régions saines, soit au contraire de diminuer la température atteinte dans des régions saines avec une incidence minimum sur la température atteinte par la tumeur à traiter.

Selon l'invention, un appareil de traitement par hyperthermie comprenant des moyens générateurs haute fréquence reliés à des électrodes susceptibles d'être couplées à un patient, est caractérisé en ce que lesdits moyens générateurs haute fréquence comportent au moins deux générateurs fonctionnant à des fréquences différentes.

Avec plusieurs générateurs fonctionnant chacun à une fréquence distincte de celle des autres générateurs, les phases des ondes électromagnétiques produites par ces différents générateurs ne sont pas corrélées entre elles. Il est alors possible d'une part de définir une zone chauffée par les énergies hautes fréquences produites par chacun des générateurs, sans que cette zone chauffée ne présente les perturbations dûes aux effets d'interférence précédemment mentionnés ; il est possible d'autre part, en augmentant ou en réduisant la puissance de l'un des générateurs, d'augmenter ou de diminuer la puissance dissipée dans une partie bien définie de la région chauffée.

L'invention sera mieux comprise grâce à la description qui suit, faite à titre d'exemple non limitatif, et aux quatre figures annexées parmi lesquelles :

-la figure 1 représente de manière - schématique un appareil d'hyperthermie conforme à l'invention ;

-la figure 2 représente l'appareil de la figure 1 perfectionné par des filtres ;

-la figure 3 montre une variante de l'appareil représenté à la figure 2 ;

-la figure 4 illustre une possibilité de connexion d'électrodes montrées aux figures 1 à 3.

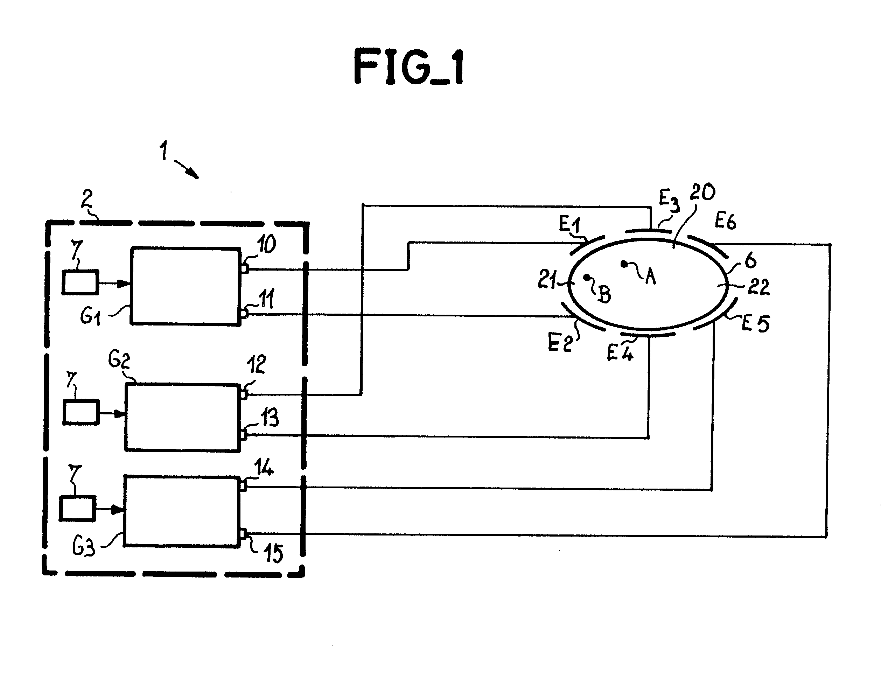

La figure 1 montre de manière schématique un appareil d'hyperthermie 1 selon l'invention, comportant des moyens générateurs 2 d'ondes haute fréquence.

Selon une caractéristique de l'invention, les moyens générateurs 2 doivent comporter au moins deux générateurs G1, G2 fonctionnant chacun à une fréquence F1, F2 différentes l'une de l'autre. Dans l'exemple non limitatif décrit, et pour mieux illustrer les possibilités de l'invention, les moyens générateurs 2 comportent en plus un troisième générateur G3 fonctionnant à une troisième fréquence F3 différente des première et seconde fréquences F1, F2.

Les fréquences F1, F2, F3 doivent avoir des valeurs différentes les unes des autres et suffisamment éloignées les unes des autres, de manière à éviter que les générateurs G1, G2, G3 débitant dans la charge représentée par un patient 6, ne tendent à un entraînement mutuel en fréquence, susceptible de conduire à une corrélation de phases entre les ondes électromagnétiques délivrées par les générateurs G1, G2, G3. Aussi, les fréquences F1, F2, F3 doivent être différentes, mais aussi sans relation simple entre elles comme dans le cas par exemple, où elles constitueraient chacune une fréquence harmonique d'une même fréquence fondamentale.

Chaque générateur G1, G2, G3 est d'un type en lui-même connu, produisant une onde électromagnétique, sous une puissance de l'ordre de 500 W par exemple, ajustable grâce à un moyen de commande de puissance 7 propre à chaque générateur G1, G2, G3. Les générateurs peuvent fonctionner à une fréquence comprise par exemple entre 100 KHertz et plusieurs dizaines de MHertz. Dans l'exemple non limitatif décrit, le premier générateur G1 fonctionne à une fréquence F1 égale à 5,5 MHZ, le second générateur G2 fonctionne à une fréquence F2 égale à 8,5 MHZ, le troisième générateur G3 fonctionne à une troisième fréquence F3 égale à 13 MHZ :

-une première et une seconde sortie 10, 11 du premier générateur G1 sont reliées respectivement à une première et une seconde électrodes E1, E2, formant une première paire d'électrodes ;

-le second générateur G2 comporte une troisième et une quatrième sorties 12, 13 reliées respectivement à une troisième et quatrième électrodes E3, E4, formant une seconde paire d'électrodes ;

-une cinquième et une sixième sorties 14, 15 du troisième générateur G3, sont reliées respectivement à une cinquième et une sixième électrodes E5, E6, formant une troisième paire d'électrodes.

Dans l'exemple non limitatif de la description, les électrodes E1 à E6 d'une même paire d'électrodes sont appliquées au patient 6 de manière à être disposées sensiblement en vis à vis de part et d'autre d'une région 20 à chauffer ; les électrodes E3, E4 de la deuxième paire étant par exemple centrales et les électrodes E1, E2 de la première paire et les électrodes E5, E6 de la troisième paire étant respectivement vers une première et une seconde extrémité 21, 22 de la région 20 à chauffer.

En considérant dans la région 20 à chauffer un point A situé entre les électrodes E2, E4 de la deuxième paire et plutôt du côté de la première extrémité 21 de la région 20 à chauffer, la puissance dissipée dans un petit volume entourant le point A est dûe, pour la part la plus importante à l'énergie fournie par le second générateur G2, et pour une plus faible part, par l'énergie fournie par le premier générateur G1 ; la contribution du troisième générateur G3 étant encore plus faible, par le fait que les cinquième et sixième électrodes E5, E6 correspondantes sont plus éloignées du point A. La puissance dissipée dans la région A est en fait égale à la somme des puissances fournies dans cette région A par chacun des générateurs G1, G2, G3, et, en modifiant les commandes de puissance 7 associées respectivement à chacun des générateurs G1, G2, G3, on peut faire varier la puissance dissipée en A, sans trop modifier la puissance fournie en un second point B ; le second point B étant par exemple situé vers la première extrémité 21 entre les première et seconde électrodes E1, E2 du premier générateur G1, sa température est peu affectée par une modification de puissance du troisième générateur G3.

Ceci constitue une amélioration importante par rapport à l'art antérieur comportant soit un unique générateur, soit plusieurs générateurs fonctionnant à une fréquence unique ; dans ce dernier cas, un échange d'énergie haute fréquence s'effectuant entre les électrodes appartenant à des générateurs différents, en fonction de la phase relative de ces générateurs, il est difficile de prévoir avec précision les répercussions entraînées par la modification de puissance d'un seul générateur.

Il est ainsi possible, avec un appareil d'hyperthermie selon l'invention, de modifier et d'ajuster de manière simple, au cours d'un traitement, la distribution de la puissance dissipée ; des profils de température de plus en plus complexes pouvant être obtenus en augmentant le nombre de générateurs G1, G2, G3 fonctionnant à des fréquences différentes.

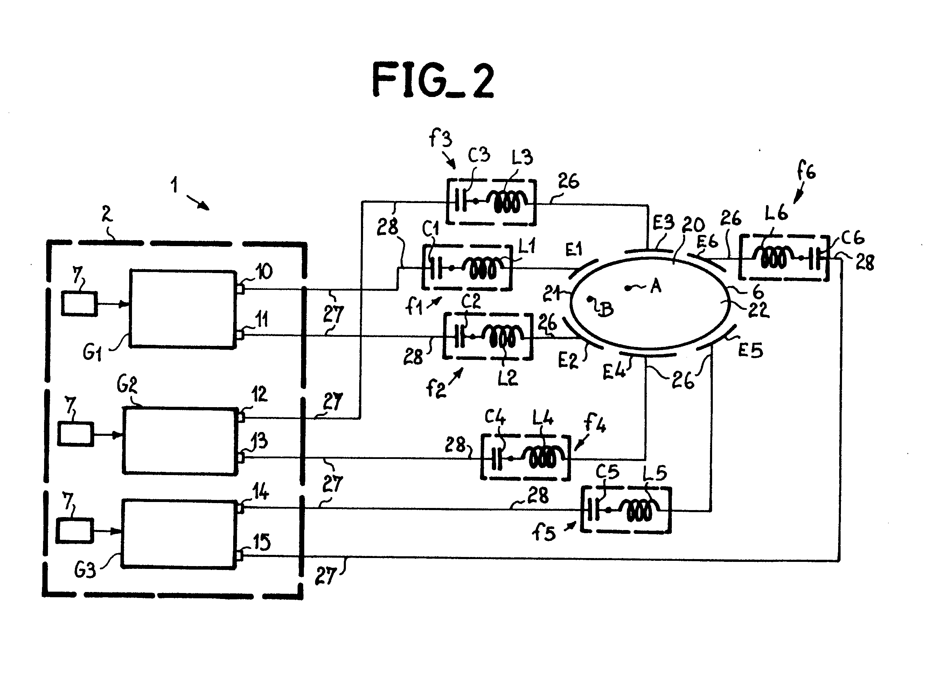

La figure 2 représente une même configuration générale de l'appareil d'hyperthermie 1 selon l'invention que dans la figure 1, mais comportant en outre des filtres f1, f2, ..., f6 placés en série entre au moins une des sorties des générateurs G1, G2, G3, et les électrodes E1, E2, ..., E6.

Les filtres f1, ..., f6 constituent des filtres passe bande accordés sur la fréquence F1, F2, F3 de fonctionnement des générateurs G1, G2, G3 auxquels ils sont reliés.

Dans l'exemple non limitatif décrit, chaque filtre f1 à f6 est relié d'une part par une première extrémité 26 respectivement aux électrodes E1 à E6, chaque filtre f1 à f6 étant relié d'autre part par une extrémité opposée 28, aux sorties correspondantes des générateurs G1, G2, G3 :
-la première sortie 10 du premier générateur G1 est reliée par une liaison 27 au premier filtre f1, la seconde sortie 11 étant reliée par une liaison 27 au second filtre f2 ;
-la troisième sortie 12 du second générateur G2 est reliée au troisième filtre f3, la quatrième sortie 13 étant reliée par une liaison 27 au quatrième filtre f4 ;
-la cinquième sortie 14 du troisième générateur G3 est reliée par une liaison 27 au cinquième filtre f5, la sixième sortie 15 étant reliée au sixième filtre f6.

Cette mise en série des filtres f1, ..., f6 constitue une caractéristique importante de l'invention, qui est rendue possible du fait que les générateurs G1, G2, G3 fonctionnent à des fréquences f1, f2, f3 différentes, et qui permet ainsi à chacun de ces générateurs de présenter une impédance élevée vis à vis des deux autres générateurs. Il en résulte que la puissance fournie par chacun des générateurs n'est dissipée que dans la charge 6 que constituent les tissus biologiques, c'est-à-dire la région à chauffer 20, et non partiellement dans les autres générateurs comme c'est le cas dans l'art antérieur ; ceci permettant en outre de mieux maîtriser encore la distribution de la puissance dissipée, en rendant les générateurs G1, G2, G3 davantage indépendants les uns des autres.

Dans l'exemple non limitatif de la description, les filtres f1 à f6 sont constitués chacun par une inductance L1 à L6 et une capacité C1 à C6 placées en série, et dont les valeurs sont choisies de manière que :
-les premier et second filtres f1, f2 résonnent chacun à la première fréquence F1 du premier générateur G1 ;
-que les troisième et quatrième filtres f3, f4 résonnent à la seconde fréquence F2 du second générateur G2 ;
-que les cinquième et sixième filtres f5, f6 résonnent à la troisième fréquence F3 du troisième générateur G3.

Il est à remarquer que dans l'exemple non limitatif décrit, les première, seconde et troisième fréquences F1, F2, F3 sont respectivement telles que la seconde fréquence F2, qui est intermédiaire entre les deux autres fréquences F1, F3, représente sensiblement la moyenne géométrique des valeurs de ces deux autres fréquences F1, F3, soit : $F1.F3 = F2^2$ ; ceci étant favorable à une réalisation simple des filtres f1, ..., f6.

On observe en pratique qu'un découplage satisfaisant des générateurs G1, G2, G3, est obtenu quand chacun des filtres f1 à f6 présente, en série avec la résistance que constitue le tissu biologique et pour les fréquences F1, F2, F3 des générateurs G1, G2, G3 autres que sa fréquence de résonance, une impédance sensiblement égale ou supérieure à dix fois la résistance du tissu biologique.

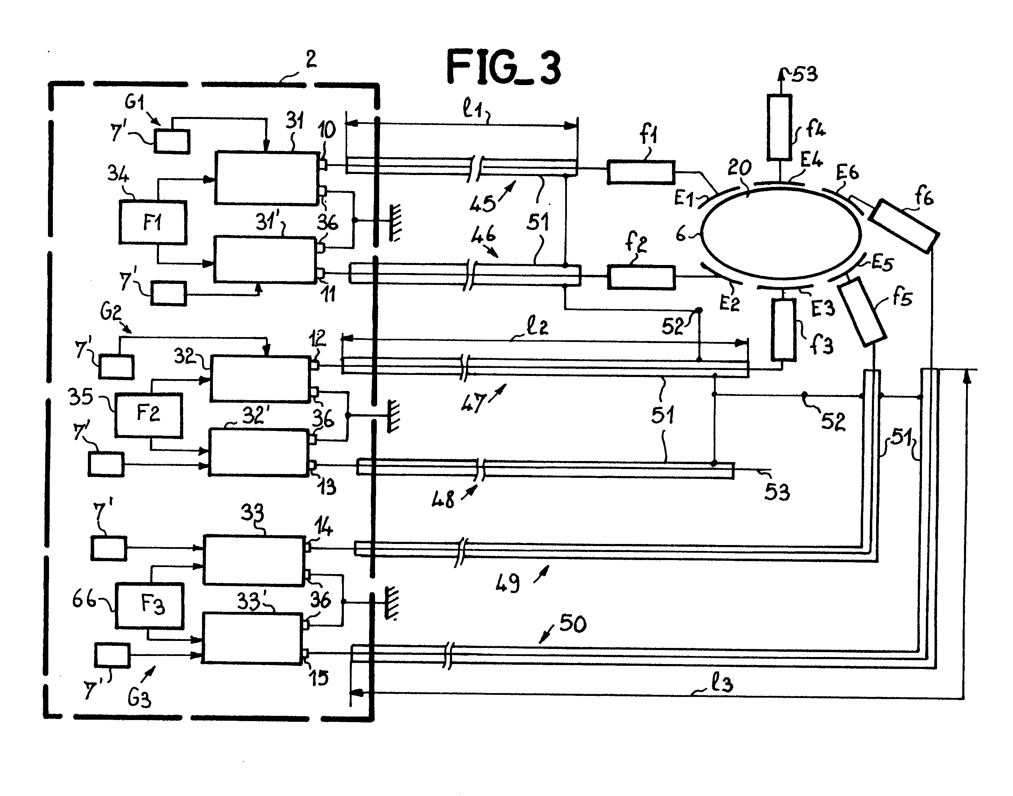

La figure 3 représente une version de l'appareil d'hyperthermie selon l'invention, dans laquelle chacun des générateurs G1, G2, G3 comporte deux demi-générateurs ou amplificateurs de sortie respectivement 31, 31' et 32, 32' et 33, 33', recevant respectivement les signaux d'un même oscillateur 34, 35, 66 ; chacun des oscillateurs 34, 35, 66 fonctionnant à la fréquence F1, F2, F3 du générateur G1, G2, G3 auquel il appartient. Chaque amplificateur de sortie 31, 31', ..., 33, 33' comporte un moyen de réglage de puissance 7' séparé, permettant de régler le niveau de puissance de l'onde produite par chacun des ces amplificateurs de sortie, les amplificateurs de sortie d'un même générateur fournissant des signaux en opposition de phase par rapport à une masse commune, selon le schéma décrit ci-après :
-le premier amplificateur 31 et le second amplificateur 31' appartenant au premier générateur G1, comportent chacun une sortie de masse 36 reliée à la masse ; une sortie 10 du premier amplificateur 31 constituant la première sortie 10 précédemment mentionnée, étant reliée à la première électrode E1 ; une sortie 11 du second amplificateur 31′ constituant la seconde sortie 11 précédemment mentionnée, étant reliée à la seconde électrode E2 ;

-le troisième et quatrième amplificateurs 32, 32′ appartenant au second générateur G2, comportent une sortie de masse 36 reliée à la masse ; une sortie 12 du troisième amplificateur 32 constituant la troisième sortie 12 précédemment mentionnée, étant reliée à la troisième électrode E3 ; une sortie 13 du quatrième amplificateur 32′ constituant la quatrième sortie 13 précédemment mentionnée, étant reliée à la quatrième électrode E4 ;

-les cinquième et sixième amplificateurs 33, 33′ appartenant au troisième générateur G3, comportent chacun une sortie de masse 36 reliée à la masse ; une sortie 14 du cinquième amplificateur constituant la cinquième sortie 14 précédemment mentionnée, étant reliée à la cinquième électrode E5 ; une sortie 15 du sixième amplificateur 33′ constituant la sixième sortie 15 précédemment mentionnée, étant reliée à la sixième électrode E6.

Dans l'exemple non limitatif décrit, les sorties 10 à 15 des générateurs G1, G2, G3 sont reliées aux électrodes E1 à E6 par l'intermédiaire des filtres f1 à f6, et les liaisons 27 sont remplacées par des câbles coaxiaux ou lignes coaxiales respectivement 45, 46, 47, 48, 49, 50. Les conducteurs extérieurs 51 de ces câbles coaxiaux sont reliés à la masse commune aux générateurs G1, G2, G3, et sont réunis également entre eux en un point commun 52 du côté des électrodes E1 à E6. L'impédance présentée par les tissus biologiques à chaque électrode E1 à E6 par rapport au point commun 52 dépend pour une part des dimensions de ces électrodes et de la nature des tissus biologiques ; typiquement cependant, en hyperthermie profonde, cette impédance est essentiellement résistive, de l'ordre de 10 OHMS. En supposant que les générateurs G1, G2, G3 comportent comme il est classique, des impédances de sortie de 50 OHMS, on peut avantageusement utiliser des câbles coaxiaux 45 à 50 d'impédance caractéristique de 25 OHMS et dont les longueurs l1, l2, l3 correspondent au quart de la longueur d'onde ($\lambda/4$) de la première ou seconde ou troisième fréquence F1, F2, F3 de l'onde H.F transmise ; l'impédance de 25 OHMS pouvant être obtenue par exemple en utilisant deux câbles coaxiaux montés en parallèles, et ayant chacun une impédance caractéristique de 50 OHMS.

Un avantage supplémentaire important de cette configuration réside en ce qu'elle permet de réduire le rayonnement parasite à des valeurs très faibles, et que l'ensemble des éléments étant à basse impédance, il est possible de parfaire les adaptations de manière simple.

Un autre avantage important réside dans le fait que les générateurs G1, G2, G3 étant séparés chacun en deux parties formées par les amplificateurs 31, 31′, ..., 33, 33′, leur puissance peut être partagée également en deux parties, produisant une puissance de 250 Watts chacune par exemple. Dans ces conditions chacun de ces amplificateurs peut être constitué par des semiconducteurs actifs présentant une basse impédance, et qui sont ainsi plus faciles à adapter à des charges formées par des tissus biologiques que dans le cas de générateur comportant des tubes électroniques tels qu'utilisés lorsqu'il s'agit de puissances élevées.

Pour plus de clarté de la figure 3, la connexion entre le quatrième filtre f4 de la quatrième électrode E4 et une extrémité 53 du quatrième câble coaxial 48, appartenant au second générateur G2, n'a pas été représentée.

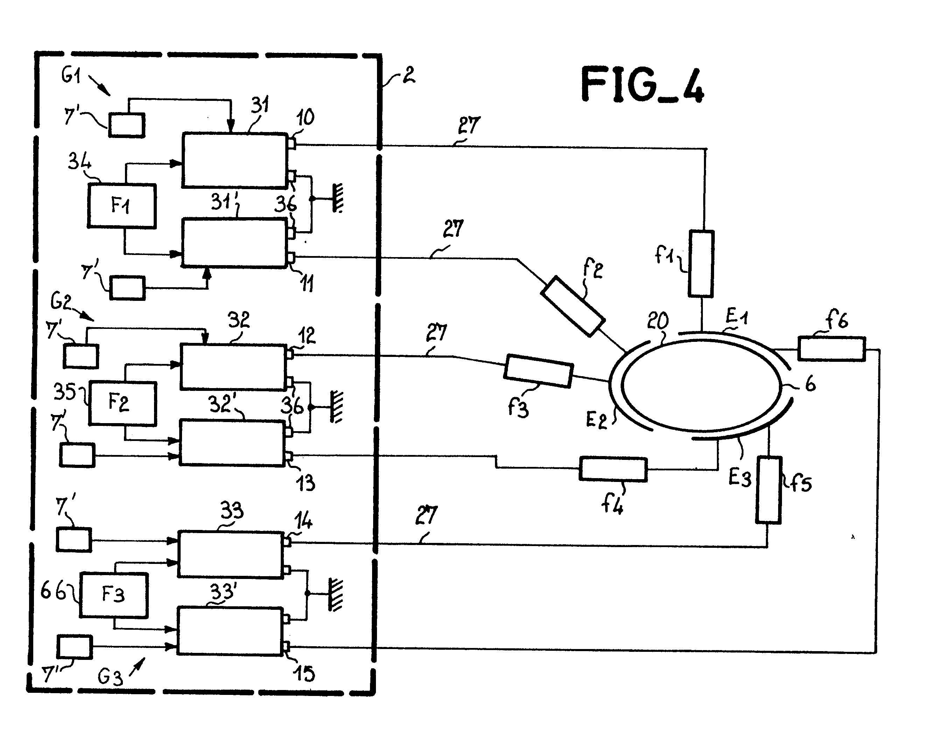

La figure 4 montre une variante de l'invention illustrant les possibilités de connexion des générateurs G1, G2, G3 aux électrodes, dans une configuration utilisant un nombre minimum d'électrodes E1, E2, E3, tout en permettant, comme dans les exemples précédents, de modifier la distribution de la puissance dissipée.

Dans l'exemple non limitatif décrit, les générateurs G1, G2, G3 sont représentés selon la version à deux amplificateurs, mais pourraient également être constitué chacun, ou l'un d'entre eux, par un générateur unique ainsi que représenté sur les figures 1 et 2 ; pour la clarté de la figure 4, les générateurs G1, G2, G3 sont reliés aux électrodes par l'intermédiaire de liaisons simples 27, mais pourraient être reliés tout aussi bien par les câbles coaxiaux 45 à 50 précédemment mentionnés.

Dans cette variante de l'invention, la région 20 à chauffer est entourée par trois électrodes E1, E2, E3 ayant une section sensiblement en arc de cercle, chacune de ces électrodes étant commune à deux générateurs G1, G2 ou G1, G3 ou G2, G3, selon l'exemple non limitatif du schéma décrit ci-après :

-la première électrode E1 est reliée d'une part à la première sortie 10 du premier générateur G1 par l'intermédiaire du premier filtre f1, et reliée d'autre part à la sixième sortie 15 du troisième générateur G3, par l'intermédiaire du sixième filtre f6 ;

-la seconde électrode E2 est reliée d'une part à la seconde sortie 11 du premier générateur G1 par l'intermédiaire du second filtre f2, et reliée d'autre part à la troisième sortie 12 du second générateur G2 par l'intermédiaire du troisième filtre f3 ;

-la troisième électrode E3 est reliée d'une part à la quatrième sortie 13 du second générateur G2 par l'intermédiaire du quatrième filtre f4, et reliée d'autre part à la cinquième sortie 14 du troisième générateur G3 par l'intermédiaire du cinquième filtre f5.

Les électrodes E1, E2, E3 s'étendent le long de la zone 20 à chauffer, selon une longueur (non représentée) perpendiculaire au plan de la figure, et l'échange d'énergie entre les électrodes E1, E2, E3 s'effectue sur cette longueur essentiellement selon des plans perpendiculaires à cette longueur, c'est-à-dire parallèle au plan de la figure.

Cette configuration permet d'ajuster avec un minimum d'électrodes la distribution de puissance et par suite la température dans la région 20 à chauffer, la contribution de chacun des générateurs G1, G2, G3, au niveau de chaque électrode E1, E2, E3 étant ajustable de manière indépendante, sans réaction sur les autres générateurs, et sans produire dans la région à chauffer 20 d'autres effets que ceux directement liés à la modification de puissance exercée sur l'un des générateurs.

L'exemple de la figure 4 montre qu'il est possible de connecter une électrode, la troisième électrode E3 par exemple, à deux sorties 13, 14 ou plus de générateurs G2, G3 différents.

## Revendications

1. Appareil de traitement par hyperthermie comprenant des moyens générateurs (2) haute fréquence reliés à des électrodes (E1, E2, ..., E6) susceptibles d'être couplées à un patient (6), caractérisé en ce que lesdits moyens générateurs - (2) comportent au moins deux générateurs (G1, G2) fonctionnant à des fréquences (F1, F2) différentes de valeurs suffisamment éloignées l'une de l'autre pour éviter un entraînement mutuel en fréquence des générateurs (G1, G2), les fréquences (F1,F2) de fonctionnement des générateurs (G1,G2) ayant des valeurs telles qu'elles ne constituent pas une fréquence harmonique d'une même fréquence fondamentale, et en ce que chacun desdits générateurs comporte deux sorties (10,11,12,13) reliées chacune à une électrode (E1-E2, E3-E4), au moins une desdites sorties (10,11,12,13) d'au moins un générateur - (G1,G2) étant reliée à ladite électrode par l'intermédiaire d'un filtre passe-bande (f1,f2,f3,f4), la fréquence de résonance dudit filtre (f1,f2,f3,f4) correspondant sensiblement à la fréquence (F1,F2) de fonctionnement du générateur (G1,G2) auquel il est relié.

2. Appareil d'hyperthermie selon la revendication 1, caractérisé en ce que lesdits générateurs - (G1, G2) comportent chacun un moyen de réglage de puissance (7, 7a).

3. Appareil d'hyperthermie selon la revendication 1, caractérisé en ce que au moins un desdits générateurs (G1, G2) comporte deux amplificateurs de sortie (30, 30') fonctionnant à la même fréquence (F1, F2).

4. Appareil d'hyperthermie selon la revendication 3, caractérisé en ce que lesdits amplificateurs de sortie (31, 31') comportent chacun un moyen de réglage de puissance (7a).

5. Appareil d'hyperthermie selon la revendication 4, caractérisé en ce que chaque amplificateur de sortie (31, 31') comporte une sortie (10,11) reliée à une électrode (E1, E2), et une sortie (36) reliée à la masse.

6. Appareil d'hyperthermie selon l'une des revendications précédentes, caractérisé en ce que lesdits générateurs (G1, G2) sont reliés auxdites électrodes (E1, E2, E3, E4) par l'intermédiaire de lignes coaxiales (45, 46, 47, 48) dont la longueur - (11, 12, 13) est sensiblement le quart de la longueur d'ondes correspondant à la fréquence (F1, F2) du générateur (G1, G2) auxquels elles sont reliées.

7. Appareil d'hyperthermie selon la revendication 6, caractérisé en ce que lesdites lignes coaxiales (45, 46, 47, 48) ont une impédance caractéristique sensiblement égale à 25 OHMS.

8. Appareil d'hyperthermie selon l'une des revendications précédentes, caractérisé en ce que lesdits générateurs (G1, G2) ont au moins une sortie (11, 12) reliée à une même électrode (E2).

**FIG_1**

FIG_2

# FIG_3

0 232 638

FIG_4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-3 147 014 (DATRON ELECTRONIC PICHL UND SCHULTE KG) * Figures 7,8; page 21, ligne 1 - page 24, ligne 12 * | 1,2,4 5 | A 61 N 1/32 |
| | --- | | |
| A | FR-A- 818 881 (LE MATERIEL TELEPHONIQUE) * Figures 1-5; page 2, ligne 25 - page 3, ligne 71 * | 1 | |
| | --- | | |
| A | US-A-3 800 802 (BERRY et al.) * Figure 1a; colonne 3, ligne 58 - colonne 5, ligne 53 * | 1,2,4 6 | |
| | --- | | |
| A | EP-A-0 119 148 (C.O.F.R.E.M. INTERNATIONAL SA) * Résumé; figure 1; page 3, ligne 15 - page 4, ligne 17 * | 1,2,4 | DOMAINES TECHNIQUES RECHERCHES (Int. Cl.4) |
| | --- | | A 61 N |
| A | FR-A-1 083 425 (SOCIETE PARISIENNE POUR L'EXPLOITATION DES ETABLISSEMENTS G. DUFLOT) * Figure 1; page 2, colonne de droite, lignes 28-47 * | 1-3,8 | |
| | ----- | | |

Le présent rapport de recherche a été établi pour toutes les revendications

| Lieu de la recherche | Date d'achèvement de la recherche | Examinateur |
|---|---|---|
| LA HAYE | 17-03-1987 | CHEN A.H. |